# EUROPEAN PATENT SPECIFICATION

(11) **EP 4 347 570 B1**
(45) Date of publication and mention of the grant of the patent: **12.02.2025**
(21) Application number: 22729712.4
(22) Date of filing: 20.05.2022
(51) Int. Cl.: C07D 251/24

(54) **PROCESS FOR THE PREPARATION OF BIS-RESORCINYL-TRIAZINES**
VERFAHREN ZUR HERSTELLUNG VON BIS-RESORCINYL-TRIAZINEN
PROCÉDÉ DE PRÉPARATION DES TRIAZINES DE BIS-RESORCINYLE

(30) Priority: 31.05.2021 EP 21176930
(43) Date of publication of application: 10.04.2024
(73) Proprietor: DSM IP Assets B.V., 6221 BE Maastricht (NL); Suqian Cosmos Chemical Co.,Ltd, Suqian, Jiangsu (CN)
(72) Inventor: HESSE, Richard, 4303 Kaiseraugst (CH); MEEUWSE, Marco, 4303 Kaiseraugst (CH); MITSCHI, Dominique, 4303 Kaiseraugst (CH); ZWEIFEL, Theodor, 4303 Kaiseraugst (CH)
(74) Representative: dsm-firmenich IP
(86) International application number: PCT/EP2022/063787
(87) International publication number: WO 2022/253599

(56) References cited:
- WO-A1-2016/184764
- WANG ET AL.: "Synthesis, Spectra, and Theoretical Investigations of 1,3,5-Triazines Compounds as Ultraviolet Rays Absorber Based on Time-Dependent Density Functional Calculations and three-Dimensional Quantitative Structure-Property Relationship", JOURNAL OF FLUORESCENCE, vol. 28, no. 2, 2 May 2018 (2018-05-02), pages 707 - 723, XP036513801, ISSN: 1053-0509, [retrieved on 20180502], DOI: 10.1007/S10895-018-2235-2
- JIANG ET AL.: "Improved synthesis of 6-(4-methoxyphenyl)-2,4-dichloro-1,3,5-triazine and 2,4-bis resorcinyl-substituted UV light absorbing derivatives", JOURNAL OF CHEMICAL RESEARCH, vol. 11, January 2008 (2008-01-01), pages 664 - 665, XP009185586, ISSN: 1747-5198, DOI: 10.3184/030823408X375142

## Description

The invention relates to an improved process for the manufacture of bis-resorcinyl triazines of formula (I), wherein R¹ is C₁-C₁₈alkyl or C₂-C₁₈alkenyl.

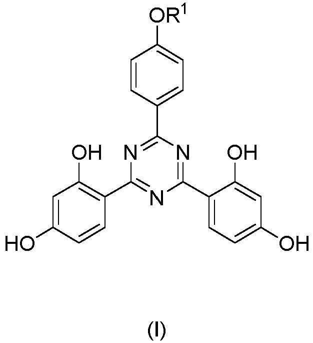

Triazines are highly effective UV-absorbers which may, for example, be used as light screening agents in cosmetic products. A particular suitable triazine is Bis-Ethylhexyloxyphenol Methoxyphenyl Triazine (BEMT) which is generally prepared using a bis-resorcinyl triazines of formula (I) as intermediate.

The preparation of said intermediate is well known in the art and e.g. disclosed in US-5955060 or in WO-2016184764. The preparation encompasses the reaction of cyanuric chloride with a phenyl magnesium bromide compound in a Grignard reaction to a dichlorotriazine. The two resorcinyl groups are then introduced by a Friedel-Crafts arylation with resorcinol in the presence of a Lewis acid, in particular an aluminium halide to form a bis-resorcinyltriazine of formula (I).

However, the processes of the prior art have significant drawbacks, as the compounds of formula (I) exhibit a bad filtration performance, causing the filtration and cake washing to be the dominant process bottleneck.

Because of the continuously increasing demand for Bis-Ethylhexyloxyphenol Methoxyphenyl Triazine, the objective of the present invention was to develop a method which results in a suspension of bis-resorcinyltriazin of formula (I) with better filtration properties. It has furthermore been found, that the process according to the present invention reduces the need of additional washing steps while still providing high purity products, thus affording further economic advantages, in particular in large scale production.

Therefore, in a first aspect the present invention relates to a process for the preparation of bis-resorcinyl triazines of formula (I) (in the following abbreviated as BRT-(I)), said process comprising the step of reacting dichlorotriazine of formula (II) in at least one aromatic solvent, 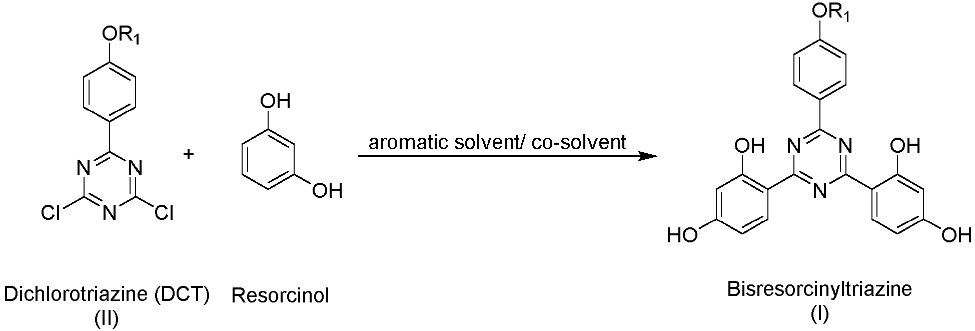
wherein R¹ is a C₁-C₁₈alkyl group or a C₂-C₁₈ alkenyl group,
with resorcinol in the presence of a Lewis acid and a co-solvent (Friedel-Crafts arylation), characterized in that after the reaction is complete
   (i) the reaction mixture is added to water while the aromatic solvent is concomitantly distilled off to obtain an aqueous suspension of the BRT-(I), followed by,
   (ii) addition of an organic solvent, which is able to dissolve BRT-(I) in an amount of at least 1 wt.-% and which leads to the formation of a biphasic system consisting of (a) a solution of BRT-(I) in said solvent (referred to as BRT-(I)-solution) and (b) water, followed by
   (iii) separation of said BRT-(I)-solution from the water phase and optionally washing the BRT-(I)-solution with water, and
   (iv) addition of the thus obtained BRT-(I)-solution to water while the organic solvent is concomitantly distilled off to obtain an aqueous suspension of BRT-(I).

Filtration of the aqueous suspension obtained in step (iv) exhibits shorter filtration times compared to the processes according to the prior art. Furthermore, only small quantities of (<1%) BRT-(I) remain in the water phase removed in step (iii) leading to excellent yields.

Thus, in a preferred embodiment, the process according to the present invention comprises a step (v) following step (iv), which step (v) comprises the filtration of the aqueous suspension of BRT-(I) obtained in step (iv), to obtain solid BRT-(I) and optionally further drying of the said solid BRT-(I). It is well understood that the filtration as well as the drying of the filter cake, i.e. the solid BRT-(I) obtained after filtration can be performed by conventional techniques known in the art such as e.g. filtration by suction, pressurized filtration or centrifugation and drying e.g. by applying heat and/or vacuum.

In contrast to the processes of the prior art, the product obtained in step (v) can directly be alkylated, without any further washing and/ or purification e.g. for the conversion into Bis-Ethylhexyloxyphenol Methoxyphenyl Triazine. Said conversion is generally carried out by etherification of the free p-hydroxyl groups of the resorcinyl residues with an alkyl halogenide as e.g. outlined in WO2016184766.

Thus, in a further embodiment, the process according to the present invention comprises in a subsequent step the reaction of the solid BRT-(I) obtained in step (v), without any further purification (e.g. washing of the filter cake), with an alkyl halogenide, preferably with ethylhexyl chloride, most preferably in the presence of a base such as in particular sodium carbonate, sodium phosphate and sodium hydrogen carbonate as outlined in WO2016184766 which is included herein by reference. Most preferably Bis-Ethylhexyloxyphenol Methoxyphenyl Triazine is prepared, i.e. the BRT-(I) is 4,4'-(6-(4-methoxyphenyl)-1,3,5-triazine-2,4-diyl)bis(benzene-1,3-diol) and the alkylhalogenide is ethylhexyl chloride.

Examples of C₁-C₁₈alkyl or C₂-C₁₈alkenyl are branched or unbranched alkyl, respectively alkenyl residues such as methyl, ethyl, n-propyl, 1-methylethyl, n-butyl, 1-methylpropyl-, 2-methylpropyl, 1,1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 2-ethylhexyl, n-octyl, n-nonyl, n-decyl, n-undecyl, n-dodecyl, prenyl, 2-propenyl and 3-butenyl.

In all embodiments of the present invention R¹ is preferably a C₁-C₅alkyl residue, more preferably a C₁-C₂alkyl residue, most preferably a methyl residue.

The Friedel Crafts arylation is well known to a person in the art and can be performed according to standard processes disclosed in the prior art such as e.g. outlined in US-5955060 or in WO-2016184764.

Particularly suitable aromatic solvents to be used in the Friedel-Crafts arylation according to the present invention are benzol, toluene or xylene, toluene being particularly preferred.

The term co-solvents as used herein refers to chemicals characterized by their ability to be miscible with toluene. Particular suitable co-solvents in all embodiments of the present invention encompass sulfolane, benzonitrile, chlorobenzene, nitrobenzene, acetonitrile and pivalonitrile as well as mixtures thereof. In all embodiments of the present invention it is preferred that only one co-solvent is used. Preferably the one co-solvent is selected from the group consisting of nitrobenzene, pivalonitrile and benzonitrile. Most preferably the co-solvent is nitrobenzene or benzonitrile.

Suitable Lewis acids encompass aluminium halides as well as magnesium halides. In all embodiments of the present invention the preferred Lewis acid is aluminium trichloride (AlCl₃).

In a particular advantageous embodiment according to the present invention R¹ is a methyl group, the aromatic solvent is toluene and the co-solvent is benzonitrile and the Lewis acid is aluminum trichloride.

In all embodiments of the present invention, the amount of resorcinol is preferably at least 2 mol-equivalents with respect to the dichlorotriazine of formula (II). Preferably, a slight excess of resorcinol is used. Most preferably the amount of resorcinol is selected in the range of 2 to 2.5 mol-equivalents, with respect to the dichlorotriazine of formula (II).

In all embodiments of the present invention, the amount of the co-solvent is preferably selected in the range of 0.5 to 10, more preferably in the range of 1 to 6, most preferably in the range of 2 to 5 mol-equivalents, with respect to the dichlorotriazine of formula (II).

In all embodiments of the present invention, the amount of the Lewis acid is preferably selected in the range of 0.5 to 7, more preferably in the range of 0.75 to 5, most preferably in the range of 1 to 3 mol-equivalents, with respect to the dichlorotriazine of formula (II).

In all embodiments of the invention, the reaction temperature of the Friedel-Crafts arylation is preferably selected in the range of 25 C to 100°C such as more preferably in the range of 50°C to 70°C and most preferably in the range of 55° to 65°C (at atmospheric pressure). It is well understood that the reaction temperature would have to be adjusted accordingly if pressure/ vacuum is applied in the process according to the present invention, which however can easily be adjusted by a person skilled in the art and is encompassed herein as well.

Thus, in another particular advantageous embodiment the amount of the co-solvent is selected in the range of 2 to 5 mol-equivalents with respect to the dichlorotriazine of formula (II), the amount of Lewis acid is selected in the range of 1 to 3 mol-equivalents with respect to the dichlorotriazine of formula (II) and the reaction temperature is selected in the range of 55° to 65°C (at atmospheric pressure).

The organic solvent which is able to dissolve the BRT-(I) in amount of at least 1 wt.-% according to the present invention can easily be determined by a person skilled in the art. Suitable solvents are the ones, which form a biphasic mixture with water in presence of BRT-(I) when saturated with water and which can readily be distilled off during the addition to water in step (iv), due to their boiling point. Such solvents can easily be determined by a person skilled in the art as e.g. outlined in the examples. Preferably the boiling point is less than 100°C, either as such or as azeotrop with water. It is furthermore advantageous if said solvent exhibits a limited solubility in water in order to have less organics leave via aqueous phase and exhibit a high solvent fraction in distillate, preferably by forming a heterogeneous minimum boiling azeotrope with water.

Advantageous solvents according to the present invention encompass ethyl acetate, 1-butanol, 2-butanol, methyl-ethyl ketone, pyridine, tetrahydrofuran (THF) and methyl-tetrahydrofuran (methyl-THF), tetrahydrofuran and methyl-tetrahydrofuran being particularly preferred.

Preferably, in all embodiments of the present invention, the temperature in step (ii) is selected in the range of 20 to 100°C, preferably in the range of 40 to 80°C, most preferably in the range of 50 to 75°C. In some embodiments it can be advantageous to increase the pressure which in turn results in an increase in temperature, and therefore an increase in solubility of BRT-(I) in the solvent.

In all embodiments of the present invention the ratio (w/w) of the amount of organic solvent, which is able to dissolve BRT-(I) in an amount of at least 1 wt.-% to the BRT-(I) is preferably selected in the range of 100:1 to 1:1, more preferably in the range of 75:1 to 5:1, most preferably in the range of 50:1 to 8:1. Further suitable ranges encompass 75:1 to 25:1, 100:1 to 25:1, 100:1 to 50:1, 50:1 to 5:1, 50:1 to 8:1, and 25:1 to:8:1.

In all embodiments of the present invention the ratio (w/w) of the amount of organic solvent, which is able to dissolve BRT in an amount of at least 1 wt.-% in step (ii) and the amount of water in step (iv) is preferably selected in the range of 5:1 to 0.25:1, preferably in the range of 3:1 to 0.5:1, most preferably in the range of 2:1 to 0.75:1.

Preferably, in all embodiments of the present invention, the water in step (iv) is preheated, preferably to a temperature selected in the range of 60 to 100°C, most preferably in the range of 70 to 95°C (also referred to herein as reaction temperature). It is furthermore preferred, that the dosage of the BRT-(I)-solution in step (iv) is done such, that the reaction temperature is maintained, i.e. is kept within a range of +/- 5 °C, preferably within the range of +/- 3 °C.

After the addition in step (iv), the temperature is furthermore increased by about 5-15°C in order to remove most of the solvent. Preferably, at least 95 % of the solvent is removed, more preferably at least 98 % most preferably 99 % (which amount can e.g. be determined via GC).

After step (iv) the solid BRT-(I) is preferably isolated by filtration using suitable filtration means such as suction, pressurized filtration or centrifugation and optionally drying.

Each reaction of the process according to the invention can in principle be carried out in any reactor suitable for the respective reaction type. Without restricting generality, the following are mentioned by way of example: suspension reactor, stirred tank, stirred tank cascade, tubular reactor, shell-type reactor, shell and tube reactor.

### Experimental part

### Preparation of 4,4'-(6-(4-methoxyphenyl)-1,3,5-triazine-2,4-diyl)bis(benzene-1,3-diol) (BRT-1)

In a 1L glass reactor with nitrogen inlet, mechanical stirrer, reflux condenser, thermometer and an off-gas connection to a two gas wash bottles in series filled with NaOH solution, 39 g of resorcinol and 47g of benzonitrile were added to 40g of 2,4-dichloro-6-(4-methoxyphenyl)-1,3,5-triazine in 240g toluene. The resulting solution was heated to 40°C. Then 38g AlCl₃ was added in portions. After complete addition the reaction was kept at 40°C for about 30 min and at 60°C for approx. 3 h.

In a second reactor with off-gas connection to a two gas wash bottles in series filled with NaOH solution, 500g water is heated to 95°C. Then, the reaction mixture obtained above is added slowly, in ca. 1 h into the hot water while toluene is distilled off simultaneously. After the dosing has ended, the distillation is carried until the temperature rises to 95°C to remove most of the toluene and obtain an aqueous suspension of the BRT-1 (step (i)).

500 g THF is added to the suspension, the temperature is controlled at 65°C, the BRT-1 dissolves in THF, and a two phase system is formed. The bottom (aqueous) phase is removed (step (ii) & (iii)).

In a 1 L reactor 400g water is heated to 85°C. The THF solution obtained in the previous step is slowly added into the water such that the temperature is maintained and the BRT-1 precipitates while the THF is distilled off simultaneously. After completion of the dosing of the THF/ BRT-1 solution, the distillation is carried on until a final internal temperature of 95°C. The suspension is cooled to room temperature and led, via the bottom valve, to a glass suction filter (10 cm diameter, pore size P3), where the filtration takes place under vacuum (500 mbar).

In total the advantage of the inventive process as outlined above is the reduction of the filtration time by approx. 50% (compared to the filtration directly after step (i)) and the avoidance of any further need of additional wet cake washings by the mean of water or toluene as needed in the prior art processes as e.g. outlined in the examples of WO 2016184764.

### Solvent selection

Several solvents have been tested for their suitability in the process according to the present invention. For this saturated solutions of BRT-1 (prepared as outlined above) in the solvents listed in table 1 have been prepared, then water was added, the mixture was stirred and then it was observed if phase separation occurred or not. The table outlines the suitability of different solvents for the described process, as phase separation was observed, while others did not lead to the required phase separation.

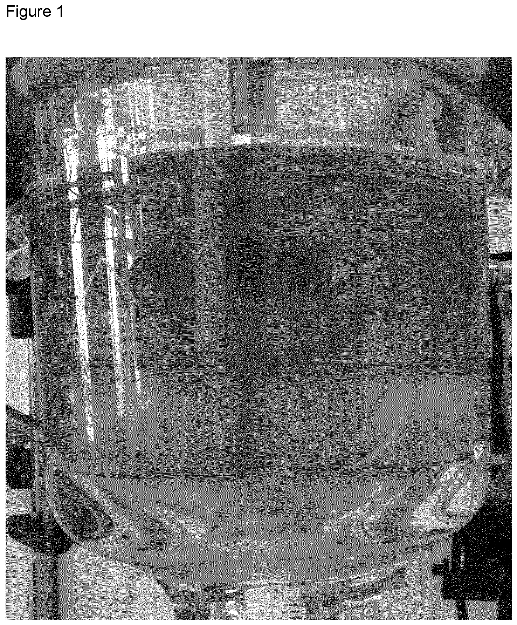

Figure 1 furthermore illustrates said suitability, depicting the phase separation of the aqueous and the THF-BRT-1 phase.

**Table 1: Suitability of solvents in the process of the invention**

| | |
|---|---|
| Toluene | not suitable |
| Isopropyl alcohol | not suitable |
| Ethyl acetate | suitable |
| Benzonitrile | not suitable |
| Acetonitrile | not suitable |
| 2-butanol | suitable |
| Methyl-THF | suitable |
| THF | suitable |

## Claims

1. Process for the preparation of bis-resorcinyl triazines of formula (I), 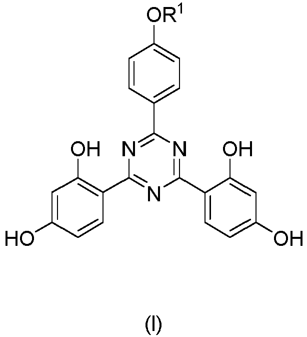
wherein R¹ is C₁-C₁₈alkyl or C₂-C₁₈ alkenyl,
said process comprising the step of reacting a bis-resorcinyl triazine of formula (II) in at least one aromatic solvent, 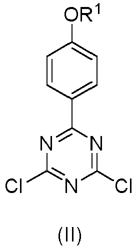
wherein R¹ is a C₁-C₁₈alkyl group or a C₂-C₁₈ alkenyl group,
with resorcinol in the presence of a Lewis acid and a co-solvent (Friedel-Crafts arylation), **characterized in that** after the reaction is complete
(i) the reaction mixture is added to water while the aromatic solvent is concomitantly distilled off to obtain an aqueous suspension of the BRT of formula (I), followed by,
(ii) addition of an organic solvent, which is able to dissolve BRT of formula (I) in an amount of at least 1 wt.-% and which leads to the formation of a biphasic system consisting of (a) a solution of BRT of formula (I) in said solvent (BRT-solution) and (b) water, followed by
(iii) separation of said BRT-solution from the water phase and optionally washing said BRT-solution with water, and
(iv) addition of the thus obtained BRT-solution to water while the organic solvent is concomitantly distilled off to obtain an aqueous suspension of a BRT of formula (I).

2. The process according to claim 1, **characterized in that** R¹ is C₁-C₂alkyl.

3. The process according to claim 1 or 2, **characterized in that** R¹ is methyl.

4. The process according to any one of claims 1 to 3, **characterized in that** the Lewis acid is aluminium trichloride.

5. The process according to any one of claims 1 to 4, **characterized in that** the co-solvent is selected from the group consisting of benzonitrile, nitrobenzene and pivalonitrile, preferably benzonitrile.

6. The process according to anyone of the preceding claims, **characterized in that** the organic solvent, which is able to dissolve BRT of formula (I) in an amount of at least 1 wt.-% is selected from the group consisting of ethyl acetate, 1-butanol, 2-butanol, pyridine, methyl-ethyl ketone, tetrahydrofuran (THF) and methyl-tetrahydrofuran (methyl-THF).

7. The process according to anyone of the preceding claims, **characterized in that** the organic solvent, which is able to dissolve the BRT of formula (I) in an amount of at least 1 wt.-% is tetrahydrofuran.

8. The process according to anyone of the preceding claims, **characterized in that** the temperature in step (ii) is selected in the range of 20 to 100°C, preferably in the range of 40 to 80°C, most preferably in the range of 50 to 75°C.

9. The process according to anyone of the preceding claims, **characterized in that** a washing step in step (ii) is performed.

10. The process according to anyone of the preceding claims, wherein the water in step (iv) is preheated, preferably to a temperature selected in the range of 60 to 100°C, most preferably in the range of 70 to 95°C.

11. The process according to anyone of the preceding claims, **characterized in that** the process comprises a subsequent filtration step (v) following step (iv), which filtration step optionally includes drying of the obtained solid BRT of formula (I).

12. The process according to claim 11, **characterized in that** the process comprises the subsequent step of alkylation of the BRT of formula (I) with an alkyl halogenide, preferably with ethylhexyl chloride.

13. The process according to claim 12, wherein said conversion is carried out in the presence of a base.

## Patentansprüche

1. Verfahren zur Herstellung von Bisresorcinyltriazinen der Formel (I), 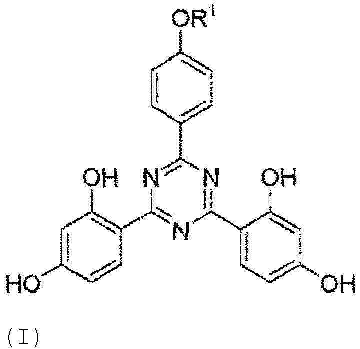
wobei R¹ C₁-C₁₈-Alkyl oder C₂-C₁₈-Alkenyl ist,
wobei das Verfahren den Schritt des Umsetzens eines Bisresorcinyltriazins der Formel (II) in mindestens einem aromatischen Lösungsmittel 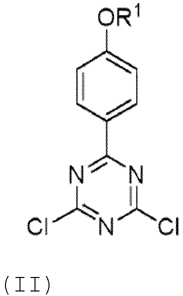
wobei R¹ für eine C₁-C₁₈-Alkylgruppe oder eine C₂-C₁₈-Alkenylgruppe steht,
mit Resorcin in Gegenwart einer Lewis-Säure und eines Cosolvens (Friedel-Crafts-Arylierung) umfasst, **dadurch gekennzeichnet, dass** nach Abschluss der Umsetzung
(i) die Reaktionsmischung zu Wasser gegeben wird, während das aromatische Lösungsmittel gleichzeitig abdestilliert wird, wobei eine wässrige Suspension des BRT der Formel (I) erhalten wird, gefolgt von
(ii) Zugabe eines organischen Lösungsmittels, das in der Lage ist, BRT der Formel (I) in einer Menge von mindestens 1 Gew.-% zu lösen, und das zur Bildung eines zweiphasigen Systems führt, das aus (a) einer Lösung von BRT der Formel (I) in dem Lösungsmittel (BRT-Lösung) und (b) Wasser besteht, gefolgt von
(iii) Abtrennung der BRT-Lösung von der Wasserphase und gegebenenfalls Waschen der BRT-Lösung mit Wasser, und
(iv) Zugabe der so erhaltenen BRT-Lösung zu Wasser, während das organische Lösungsmittel gleichzeitig abdestilliert wird, wobei eine wässrige Suspension eines BRT der Formel (I) erhalten wird.

2. Verfahren nach Anspruch 1, **dadurch gekennzeichnet, dass** R¹ für C₁-C₂-Alkyl steht.

3. Verfahren nach Anspruch 1 oder 2, **dadurch gekennzeichnet, dass** R¹ für Methyl steht.

4. Verfahren nach einem der Ansprüche 1 bis 3, **dadurch gekennzeichnet, dass** es sich bei der Lewis-Säure um Aluminiumtrichlorid handelt.

5. Verfahren nach einem der Ansprüche 1 bis 4, **dadurch gekennzeichnet, dass** das Cosolvens aus der Gruppe bestehend aus Benzonitril, Nitrobenzol und Pivalonitril, vorzugsweise Benzonitril, ausgewählt wird.

6. Verfahren nach einem der vorhergehenden Ansprüche, **dadurch gekennzeichnet, dass** das organische Lösungsmittel, das in der Lage ist, BRT der Formel (I) in einer Menge von mindestens 1 Gew.-% zu lösen, aus der Gruppe bestehend aus Essigsäureethylester, 1-Butanol, 2-Butanol, Pyridin, Methylethylketon, Tetrahydrofuran (THF) und Methyltetrahydrofuran (Methyl-THF) ausgewählt wird.

7. Verfahren nach einem der vorhergehenden Ansprüche, **dadurch gekennzeichnet, dass** es sich bei dem organischen Lösungsmittel, das in der Lage ist, BRT der Formel (I) in einer Menge von mindestens 1 Gew.-% zu lösen, um Tetrahydrofuran handelt.

8. Verfahren nach einem der vorhergehenden Ansprüche, **dadurch gekennzeichnet, dass** die Temperatur in Schritt (ii) im Bereich von 20 bis 100 °C, bevorzugt im Bereich von 40 bis 80 °C, ganz besonders bevorzugt im Bereich von 50 bis 75 °C, gewählt wird.

9. Verfahren nach einem der vorhergehenden Ansprüche, **dadurch gekennzeichnet, dass** in Schritt (ii) ein Waschschritt durchgeführt wird.

10. Verfahren nach einem der vorhergehenden Ansprüche, wobei das Wasser in Schritt (iv) vorerhitzt wird, vorzugsweise auf eine Temperatur im Bereich von 60 bis 100 °C, ganz besonders bevorzugt im Bereich von 70 bis 95 °C.

11. Verfahren nach einem der vorhergehenden Ansprüche, **dadurch gekennzeichnet, dass** das Verfahren einen nachfolgenden Filtrationsschritt (v) im Anschluss an Schritt (iv) umfasst, wobei der Filtrationsschritt gegebenenfalls das Trocknen der erhaltenen festen BRT der Formel (I) umfasst.

12. Verfahren nach Anspruch 11, **dadurch gekennzeichnet, dass** das Verfahren den nachfolgenden Schritt der Alkylierung des BRT der Formel (I) mit einem Alkylhalogenid, vorzugsweise mit Ethylhexylchlorid, umfasst.

13. Verfahren nach Anspruch 12, **dadurch gekennzeichnet, dass** die Umwandlung in Gegenwart einer Base durchgeführt wird.

## Revendications

1. Procédé de préparation de bis-résorcinyl-triazines de formule (I), 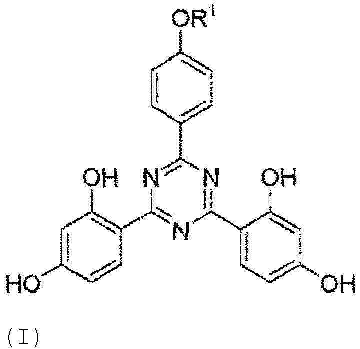
dans laquelle R¹ est C₁-C₁₈alkyle ou C₂-C₁₈ alcényle,
ledit procédé comprenant l'étape de mise en réaction d'une bis-résorcinyl-triazine of formula (II) dans au moins un solvant aromatique, 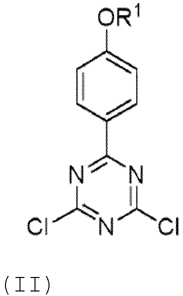
dans laquelle R¹ est un groupe C₁-C₁₈alkyle ou un groupe C₂-C₁₈ alcényle,
avec du résorcinol en présence d'un acide de Lewis et d'un co-solvant (arylation de Friedel-Crafts), **caractérisé en ce qu'**après que la réaction est terminée
(i) le mélange réactionnel est ajouté à de l'eau tout en éliminant de manière concomitante le solvant aromatique par distillation pour obtenir une suspension aqueuse du BRT de formule (I), suivi par,
(ii) l'ajout d'un solvant organique capable de dissoudre le BRT de formule (I) en une quantité d'au moins 1 % en poids et qui conduit à la formation d'un système biphasique constitué (a) d'une solution de BRT de formule (I) dans ledit solvant (solution de BRT) et (b) d'eau, suivi par
(iii) la séparation de ladite solution de BRT de la phase aqueuse et éventuellement le lavage de ladite solution de BRT avec de l'eau, et
(iv) l'ajout de la solution de BRT ainsi obtenue à de l'eau tout en éliminant de manière concomitante le solvant organique par distillation pour obtenir une suspension aqueuse d'un BRT de formule (I).

2. Procédé selon la revendication 1, **caractérisé en ce que** R¹ est C₁-C₂alkyle.

3. Procédé selon la revendication 1 ou 2, **caractérisé en ce que** R¹ est méthyle.

4. Procédé selon l'une quelconque des revendications 1 à 3, **caractérisé en ce que** l'acide de Lewis est le trichlorure d'aluminium.

5. Procédé selon l'une quelconque des revendications 1 à 4, **caractérisé en ce que** le co-solvant est choisi dans le groupe constitué par le benzonitrile, le nitrobenzène et le pivalonitrile, de préférence le benzonitrile.

6. Procédé selon l'une quelconque des revendications précédentes, **caractérisé en ce que** le solvant organique, qui est capable de dissoudre le BRT de formule (I) en une quantité d'au moins 1 % en poids, est choisi dans le groupe constitué par l'acétate d'éthyle, le 1-butanol, le 2-butanol, la pyridine, la méthyléthylcétone, le tétrahydrofuranne (THF) et le méthyl-tétrahydrofuranne (méthyl-THF).

7. Procédé selon l'une quelconque des revendications précédentes, **caractérisé en ce que** le solvant organique, qui est capable de dissoudre le BRT de formule (I) en une quantité d'au moins 1 % en poids, est le tétrahydrofuranne.

8. Procédé selon l'une quelconque des revendications précédentes, **caractérisé en ce que** la température dans l'étape (ii) est choisie dans la plage de 20 à 100 °C, de préférence dans la plage de 40 à 80 °C, le plus préférablement dans la plage de 50 à 75 °C.

9. Procédé selon l'une quelconque des revendications précédentes, **caractérisé en ce qu'**une étape de lavage dans l'étape (ii) est effectuée.

10. Procédé selon l'une quelconque des revendications précédentes, dans lequel l'eau dans l'étape (iv) est préchauffée, de préférence jusqu'à une température choisie dans la plage de 60 à 100 °C, le plus préférablement dans la plage de 70 à 95 °C.

11. Procédé selon l'une quelconque des revendications précédentes, **caractérisé en ce que** le procédé comprend une étape de filtration subséquente (v) suivant l'étape (iv), laquelle étape de filtration comprend éventuellement le séchage du BRT de formule (I) solide obtenu.

12. Procédé selon la revendication 11, **caractérisé en ce que** le procédé comprend l'étape subséquente d'alkylation du BRT de formule (I) avec un halogénure d'alkyle, de préférence avec du chlorure d'éthylhexyle.

13. Procédé selon la revendication 12, dans lequel ladite conversion est effectuée en présence d'une base.
